# EUROPEAN PATENT APPLICATION

(11) **EP 4 349 342 A1**
(43) Date of publication of application: **10.04.2024**
(21) Application number: 22926321.5
(22) Date of filing: 09.11.2022
(51) Int. Cl.: A61K 31/7004, A61P 37/00, A61P 37/06, A23L 33/15, A61K 8/60, A61Q 19/00

(54) **COMPOSITION FOR IMMUNOSUPPRESSION COMPRISING L-AHG**

(30) Priority: 10.08.2022 KR 20220099691
(71) Applicant: Kyungpook National University Industry-Academic Cooperation Foundation, Daegu 41566 (KR)
(72) Inventor: KIM, Young Ho, Gunwi-gun, Gyeongsangbuk-do 39034 (KR); PARK, Shin Young, Daegu 42509 (KR); JANG, Won Young, Daegu 41267 (KR)
(74) Representative: Reitstötter Kinzebach
(86) International application number: PCT/KR2022/017595
(87) International publication number: WO 2024/034735

(57) **Abstract**

The present disclosure relates to an immunosuppressive composition containing 3,6-anhydro-L-galactose (L-AHG) as an active ingredient, and a pharmaceutical composition for preventing or treating immunological diseases, a health functional food for preventing or ameliorating immunological diseases, and a functional cosmetic composition for preventing or ameliorating hypersensitivity skin reactions, which contain the active ingredient. The immunosuppressive composition, pharmaceutical composition, health functional food, and functional cosmetic composition containing L-AHG according to the present disclosure have an excellent effect of selectively and effectively inhibiting the activation, proliferation and differentiation of CD4 T cells, which are the main targets of immunosuppressants, and thus are very useful in the pharmaceutical industry, the food industry and the cosmetic industry.

## Description

### BACKGROUND

### 1. Technical Field

The present disclosure relates to an immunosuppressive composition containing 3,6-anhydro-L-galactose (L-AHG) as an active ingredient, and a pharmaceutical composition for preventing or treating immunological diseases, a health functional food for preventing or ameliorating immunological diseases, and a functional cosmetic composition for preventing or ameliorating hypersensitivity skin reactions, which contain the active ingredient.

### 2. Related Art

The immune system may act inappropriately in some cases. As typical examples, there are cases in which the immune system overreacts, causing hypersensitivity, or induces immune response against self-molecules due to a failure to discriminate between self and non-self molecules, resulting in autoimmune diseases. In addition, immunorejection by the recipient's immune system against allografts transplanted for therapeutic purposes can be considered as an inappropriate immune response from the viewpoint of maintenance and survival of the allografts.

Allergy or type 1 hypersensitivity occurring in the human body is mainly a reaction involving immunoglobulin E (IgE) antibodies, and when a specific antigen (allergen) binds to IgE on the surfaces of *in vivo* mast cells and basophils, various mediators causing hypersensitivity and inflammation are released from these cells. The main symptoms of hypersensitivity include sneezing, wheezing, asthma, atopic dermatitis, skin rash (hives), and the like.

Autoimmunity, in which the immune system loses its ability to discriminate between self and non-self molecules and attacks the host immunologically, causes various chronic and debilitating diseases. Major diseases caused by autoimmunity include multiple sclerosis, rheumatoid arthritis, systemic lupus erythematosus, type 1 diabetes, psoriasis, Hashimoto's thyroiditis, and acute inflammatory bowel disease [IBD; Crohn's disease/ulcerative colitis], which is a recent problem in the younger age group.

When the human body's immune system comes into encounter foreign cells or tissues, it reacts strongly to remove these foreign elements. However, in some cases, even if the patient's immune system recognizes the foreign elements and initiates an immune response to reject them, transplantation of cells, tissues or organs from other donors may be the only treatment for the patient's disease. For example, it is estimated that, in the United States alone, more than 100,000 people annually need treatment through kidney transplantation. However, the immune system attacks and eliminates any transplanted cells, tissues, or organs, which are not recognized as self, through immunorejection. Thus, immunorejection has become a serious obstacle to life-saving transplant therapy. An additional risk in transplantation is that immune cells contained within the donated tissue (e.g., a bone marrow transplant to restore immunity) recognize the new host as non-self and responds against the new host. This response, called graft-versus-host disease (GVHD), can be fatal.

Therefore, immunosuppressive drugs have been used to control inappropriate immune responses such as hypersensitivity, autoimmune diseases, immunorejection against allografts, and graft-versus-host disease, which occur in the human immune system. Immunosuppressants that are currently used include corticosteroids (prednisone), antiproliferative agents [mycophenolate mofetil (CellCept^{®})], calcineurin inhibitors [(tacrolimus (Envarsus XR^{®} or Protopic), cyclosporin (Gengraf^{®}, Neoral^{®} or Sandimmune^{®})], mTOR inhibitors [sirolimus (Rapamun^{®})], immune cell-targeting polyclonal antibodies and monoclonal antibodies, and the like. The current immunosuppressants used still fail to completely rule out side effects, leading to a predisposition to infectious diseases, cardiovascular risk, and damage to other organs such as the kidney and liver, and are also unsatisfactory in terms of efficacy. Therefore, there is a need to develop new safe and target-specific immunosuppressants with minimized side effects.

Recently, thanks to the enhancement of understanding of the human immune system and the development of biotechnology, for the treatment of patients with hypersensitivity, patients with autoimmune diseases, and organ transplant patients including stem cell or bone marrow transplant patients, who clinically require administration of immunosuppressants, various types of immunosuppressants have been used alone or in combination depending on the patient's condition and symptoms.

The target of action of typical immunosuppressants used in patients for the purpose of artificially suppressing human immune function is focused on T cells, and in fact, suppression of the function of mainly CD4 T(Th) cells among T cells is a representative mechanism of action. This is because effector Th cell-derived cytokines are importantly involved not only in the cellular immune response in which T cells play a leading role, but also in the humoral immune response in which B cell-derived antibody molecules play a leading role. In particular, in several diseases such as Sjogren's syndrome, Behcet's disease, systemic sclerosis, polymyositis, and dermatomyositis, in which cytotoxic CD4+ T cells were reported to be involved as an important cause, the inhibition of T cell activation can be an important treatment option.

Therefore, immunosuppressive effects can be well exerted by means capable of appropriately blocking the activation and proliferation of resting (G₀) Th cells and their differentiation into effector Th cells. The importance of Th cells involved in the induction and regulation of immune responses is also well evidenced by the phenomenon that immune deficiency syndrome occurs in patients with Th cell dysfunction caused by infection with the human immunodeficiency virus (HIV).

To alleviate the side effects, potential risks, or complications that are caused by the use of immunosuppressants developed to date, there is a continuing demand for the development of new immunosuppressants capable of specifically suppressing only inappropriate immune responses.

### SUMMARY

The present disclosure was made to solve the above-described problems occurring in the prior art, and an object of the present disclosure is to provide a novel immunosuppressive composition capable of effectively suppressing the activation, proliferation and differentiation of CD4 T cells, which are the main targets of immunosuppressants, a composition for preventing or treating immunological disease, a health functional food for preventing or ameliorating immunological disease, and a functional cosmetic composition for preventing or ameliorating hypersensitivity skin reactions.

In order to achieve the above objective, the present disclosure provides an immunosuppressive composition containing 3,6-anhydro-L-galactose (L-AHG) as an active ingredient.

The composition preferably inhibits the activation, proliferation or differentiation of CD4 T cells.

The composition preferably blocks G1 or S phase cell cycle progression.

The composition preferably inhibits differentiation into Th1 cells or Th2 cells.

The composition preferably inhibits the production of interferon-gamma (INF-γ), interleukin-2 (IL-2), interleukin-4 (IL-4), or interleukin-13 (IL-13).

The present disclosure also provides a pharmaceutical composition for preventing or treating immunological disease containing 3,6-anhydro-L-galactose (L-AHG) as an active ingredient.

The immunological disease is preferably selected from the group consisting of hypersensitivity disease, autoimmune disease, immunorejection, graft-versus-host disease, Sjogren's syndrome, and Behcet's disease.

The hypersensitivity disease is preferably selected from the group consisting of allergy, sneezing, wheezing, asthma, atopic dermatitis, and hives.

The autoimmune disease is preferably selected from the group consisting of multiple sclerosis, rheumatoid arthritis, systemic lupus erythematosus, type 1 diabetes, psoriasis, Hashimoto's thyroiditis, systemic lupus erythematosus, and acute inflammatory bowel disease.

The present disclosure also provides a health functional food for preventing or ameliorating immunological diseases containing 3,6-anhydro-L-galactose (L-AHG) as an active ingredient.

The immunological disease is preferably selected from the group consisting of hypersensitivity disease, autoimmune disease, immunorejection, graft-versus-host disease, Sjogren's syndrome, and Behcet's disease.

The hypersensitivity disease is preferably selected from the group consisting of allergy, sneezing, wheezing, asthma, atopic dermatitis, and hives.

The autoimmune disease is preferably selected from the group consisting of multiple sclerosis, rheumatoid arthritis, systemic lupus erythematosus, type 1 diabetes, psoriasis, Hashimoto's thyroiditis, systemic lupus erythematosus, and acute inflammatory bowel disease.

The present disclosure also provides a functional cosmetic composition for preventing or ameliorating hypersensitivity skin reactions containing 3,6-anhydro-L-galactose (L-AHG) as an active ingredient.

The hypersensitivity skin reactions are preferably selected from the group consisting of solar dermatitis, contact dermatitis, and atopic dermatitis.

The immunosuppressive composition, pharmaceutical composition, health functional food, and functional cosmetic composition containing L-AHG, according to the present disclosure have an excellent effect of selectively and effectively inhibiting the activation, proliferation and differentiation of CD4 T cells, which are the main targets of immunosuppressants, and thus are very useful in the pharmaceutical industry, the food industry and the cosmetic industry.

### BRIEF DESCRIPTION OF THE DRAWINGS

FIG. 1 shows the distribution of CD4 (T_{H}) and CD8 (T_{C}) cells in G0 T cells isolated from mouse spleen.
FIG. 2 shows the results of [³H]-thymidine incorporation assay performed to evaluate the effects of AGH, neoagarobiose (NA2), heoagarotetraose (NA4) and neoagarohexose (NA6), obtained from the enzymatic hydrolysis of agarose, against the proliferation of T cells activated with immobilized anti-CD3/anti-CD28.
FIG. 3 shows results indicating that L-AGH specifically blocks cell cycle progression in CD4 T cells. G0 T cells were treated with nocodazole 20 hours after activation thereof.
FIG. 4 shows results indicating that L-AGH blocks cell cycle progression of CD4 T cells not treated with nocodazole.
FIG. 5 shows results indicating that L-AGH treatment decreases the secretion of Th1 signature cytokines (INF-γ and IL-2) and Th2 signature cytokines (IL-4 and IL-13) secreted by T cells activated with immobilized anti-CD3/anti-CD28.

### DETAILED DESCRIPTION

Hereinafter, the present disclosure will be described in detail.

In the present disclosure, agarose saccharification was performed by an enzymatic process using a combination of recombinant GH16B β-agarase, GH50A β-agarase [Kwon et al, 2020] and GH117 α-neoagarobiose hydrolase (GH117A α-NABH) [Jang et al, 2021] derived from the freshwater agar-degrading bacterium *Cellvibrio sp.* KY-GH-1 strain (KCTC 13629BP) isolated from a non-marine environment, and then a mixture of the monomers 3,6-anhydro-L-galactose (L-AHG) and D-galactose, produced as final degradation products, was fractionated by size-exclusion chromatography using a Sephadex G-10 column, thereby finally purifying L-AHG. In order to investigate the immunosuppressive activity of the purified L-AHG, the effect of L-AHG on the activation and proliferation of resting Th cells was examined using a model in which *in vitro* activation and cell cycle of mouse resting (G0) T cells were induced. As a result, it was confirmed that resting Th cells excited the G0 phase through their activation by simultaneous stimulation of immobilized anti-CD3 bound to the T cell receptor complex and immobilized anti-CD28 bound to the T cell costimulatory receptor (CD28), and when the cell cycle progressed in the order of G1 → S → G2/M phase, the presence of L-AHG could exert an immunosuppressive effect of reducing the proliferation of T cells by inhibiting the progression of G1 and S phases of the cell cycle in activated Th cells.

Therefore, present disclosure provides an immunosuppressive composition containing 3,6-anhydro-L-galactose (L-AHG) as an active ingredient.

The active ingredient may be obtained from acid hydrolysis or enzymatic degradation of agarose.

In a preferred embodiment, the active ingredient of the present disclosure may be obtained by acid hydrolysis of agarose.

In another embodiment, the active ingredient of the present disclosure may be obtained by saccharifying agarose through an enzymatic process using a combination of recombinant GH16B β-agarase, GH50A β-agarase and GH117 α-neoagarobiose hydrolase derived from the agar-degrading bacterium *Cellvibrio sp.* KY-GH-1 strain (KCTC 13629BP), and then fractionating a mixture of the monomers 3,6-anhydro-L-galactose (L-AHG) and D-galactose, produced as final degradation products, by size-exclusion chromatography using a Sephadex G-10 column.

In another preferred embodiment, the active ingredient of the present disclosure may be obtained from the agarase-3,6-anhydro-L-galactosidase-arabinose isomerase enzyme complex product of agarose.

In addition to the several methods for obtaining L-AHG described above, the active ingredient of the present disclosure may also be obtained commercially. For example, the active ingredient may be purchased from Toronto Research Chemicals (North York, Ontario, Canada).

The composition preferably inhibits the activation, proliferation or differentiation of CD4 T cells.

The composition preferably blocks G1 or S phase cell cycle progression.

The composition preferably inhibits differentiation into Th1 cells or T2 cells.

The composition preferably inhibits the production of interferon-gamma (INF-γ), interleukin-2 (IL-2), interleukin-4 (IL-4), or interleukin-13 (IL-13).

The present disclosure also provides a pharmaceutical composition for preventing or treating immunological disease containing 3,6-anhydro-L-galactose (L-AHG) as an active ingredient.

The immunological disease is preferably selected from the group consisting of hypersensitivity disease, autoimmune disease, immunorejection, graft-versus-host disease, Sjogren's syndrome, and Behcet's disease.

The hypersensitivity disease is preferably selected from the group consisting of allergy, sneezing, wheezing, asthma, atopic dermatitis, and hives.

The autoimmune disease is preferably selected from the group consisting of multiple sclerosis, rheumatoid arthritis, systemic lupus erythematosus, type 1 diabetes, psoriasis, Hashimoto's thyroiditis, systemic lupus erythematosus, and acute inflammatory bowel disease.

The immunosuppressive composition and pharmaceutical composition containing the active ingredient of the present disclosure may be formulated and used in various forms, including oral formulations such as powders, granules, tablets, capsules, suspensions, emulsions, syrups, and aerosols, and injectable formulations such as sterile injection solutions, according to conventional methods depending on the respective purpose of use, and may be administered orally or administered through various routes, including intravenous, intraperitoneal, subcutaneous, intrarectal, and topical routes.

This pharmaceutical composition may further contain carriers, excipients or diluents. Examples of suitable carriers, excipients or diluents that may be contained include lactose, dextrose, sucrose, sorbitol, mannitol, xylitol, erythritol, maltitol, starch, gum acacia, alginate, gelatin, calcium phosphate, calcium silicate, cellulose, methyl cellulose, amorphous cellulose, polyvinyl pyrrolidone, water, methylhydroxybenzoate, propylhydroxybenzoate, talc, magnesium stearate, and mineral oil. In addition, the pharmaceutical composition of the present disclosure may further contain a filler, an anti-coagulant, a lubricant, a wetting agent, a flavoring agent, an emulsifier, a preservative, and the like.

In a preferred embodiment, solid formulations for oral administration include tablets, pills, powders, granules, capsules, etc., and these solid formulations are prepared by mixing the immunosuppressive composition or the pharmaceutical composition with one or more excipients, for example, starch, calcium carbonate, sucrose, lactose, gelatin, and the like. In addition, lubricants such as magnesium stearate and talc may be used in addition to simple excipients.

In a preferred embodiment, oral liquid formulations include suspensions, internal solutions, emulsions, syrups, etc., and may contain various excipients such as a wetting agent, a sweetening agent, a flavoring agent, and a preservative, in addition to water and liquid paraffin, which are commonly used simple diluents.

In a preferred embodiment, formulations for parenteral administration include sterilized aqueous solutions, non-aqueous solutions, suspensions, emulsions, lyophilized formulations, suppositories, and the like. Non-aqueous solutions and suspensions may contain propylene glycol, polyethylene glycol, vegetable oil such as olive oil, and injectable ester such as ethyl oleate. Injectable formulations may contain conventional additives such as a solubilizer, an isotonic agent, a suspending agent, an emulsifier, a stabilizer, a preservative, and the like.

The active ingredient of the present disclosure is administered in a pharmaceutically effective amount. As used herein, the term "pharmaceutically effective amount" refers to an amount sufficient to treat a disease at a reasonable benefit/risk ratio applicable to any medical treatment. The effective dose level of the active ingredient may be determined depending on factors, including the kind and severity of the patient's disease, the activity of the drug, sensitivity to the drug, the time of administration, the route of administration, excretion rate, and drugs used in combination with the active ingredient, as well as other factors well known in the medical field. The immunosuppressive composition or pharmaceutical composition of the present disclosure may be administered individually or in combination with other therapeutic agents, and may be administered sequentially or simultaneously with conventional therapeutic agents. The immunosuppressive composition or pharmaceutical composition may be administered in a single or multiple dosage form. It is important to administer the immunosuppressive composition or pharmaceutical composition in the minimum amount that can exhibit the maximum effect without causing side effects, in view of all the above-described factors, and this amount can be easily determined by a person skilled in the art.

In a preferred embodiment, the effective amount of the active ingredient in the immunosuppressive composition or pharmaceutical composition of the present disclosure may vary depending on the patient's age, sex and body weight. Generally, the active ingredient may be administered daily or every other day at a dose of 1 mg to 1,000 mg/kg body weight, preferably 5 mg to 100 mg/kg body weight, more preferably 10 mg to 20 mg/kg body weight, or may be administered 1 to 3 times a day at this dose. However, the dose is not intended to limit the scope of the present disclosure in any way, because the dose may increase or decrease depending on the route of administration, the severity of the disease, the patient's sex, weight and age, etc.

The immunosuppressive composition or pharmaceutical composition of the present disclosure may be administered to a subject through various routes. All modes of administration can be contemplated, and for example, the composition may be administered orally, intrarectally, or by intrarectal, intravenous, intramuscular, subcutaneous, intrauterine, intrathecal or intracerebroventricular injection.

As used herein, the term "administration" means providing a given substance to a patient by any suitable method. The immunosuppressive composition or pharmaceutical composition of the present disclosure may be administered orally or parenterally through any general route as long as it can reach the target tissue. In addition, the immunosuppressive composition or pharmaceutical composition of the present disclosure may also be administered using any device capable of delivering the active ingredient to target cells.

In the present disclosure, the term "subject" is not particularly limited, but includes, for example, humans, monkeys, cattle, horses, sheep, pigs, chicken, turkeys, quails, cats, dogs, mice, rats, rabbits or guinea pigs, and preferably refers to mammals, more preferably humans.

The present disclosure also provides a health functional food for preventing or ameliorating immunological diseases containing 3,6-anhydro-L-galactose (L-AHG) as an active ingredient.

The immunological disease is preferably selected from the group consisting of hypersensitivity disease, autoimmune disease, immunorejection, graft-versus-host disease, Sjogren's syndrome, and Behcet's disease.

The hypersensitivity disease is preferably selected from the group consisting of allergy, sneezing, wheezing, asthma, atopic dermatitis, and hives.

The autoimmune disease is preferably selected from the group consisting of multiple sclerosis, rheumatoid arthritis, systemic lupus erythematosus, type 1 diabetes, psoriasis, Hashimoto's thyroiditis, systemic lupus erythematosus, and acute inflammatory bowel disease.

The health functional food of the present disclosure may be variously used in foods and beverages that are effective in preventing and ameliorating immunological diseases. Foods containing the active ingredient of the present disclosure include various foods, for example, beverages, gums, teas, vitamin complexes, health supplement foods and the like, and may be used in the form of powders, granules, tablets, capsules or beverages.

The active ingredient of the present disclosure may generally be added in an amount of 0.001 to 10 wt% based on the total food weight. For a health beverage composition, the active ingredient may be added in an amount of 0.001 to 10 g, preferably 0.01 to 1 g, based on 100 ml of the health beverage composition.

The health functional food of the present disclosure may additionally contain food-acceptable additives, for example, natural carbohydrates and various flavoring agents, in addition to containing the compound as an essential component at the indicated percentage.

Examples of the natural carbohydrates include conventional sugars, such as monosaccharides (e.g., glucose, fructose, etc.), disaccharides (e.g., maltose, sucrose, etc.), polysaccharides (e.g., dextrin, cyclodextrin, etc.), and sugar alcohols such as xylitol, sorbitol, erythritol or the like.

Examples of the flavoring agents include natural flavoring agents such as thaumatin, rebaudioside A, or glycyrrhizin, saccharin, and synthetic flavoring agents such as aspartame. The natural carbohydrates are generally used in an amount of about 1 to 20 g, preferably about 5 to 12 g, based on 100 mL of the health functional food of the present disclosure.

In addition, the health functional food of the present disclosure may contain various nutrients, vitamins, minerals, flavoring agents such as synthetic flavoring agents and natural flavoring agents, colorants, extenders, pectic acid and its salt, alginic acid and its salt, organic acids, protective colloidal thickeners, pH adjusting agents, stabilizers, preservatives, glycerin, alcohol, carbonizing agents that are used in carbonated beverages, etc.

Additionally, the health functional food of the present disclosure may contain fruit flesh that is used for the preparation of natural fruit juice, fruit juice beverages or vegetable beverages. These components may be used individually or in combination. The content of these additives is generally selected in the range of 10 to about 50 parts by weight based on 100 parts by weight of the active ingredient of the present disclosure.

The present disclosure also provides a functional cosmetic composition for preventing or ameliorating hypersensitivity skin reactions containing 3,6-anhydro-L-galactose (L-AHG) as an active ingredient.

The hypersensitivity skin reaction is preferably selected from the group consisting of solar dermatitis, contact dermatitis, and atopic dermatitis.

The active ingredient in the cosmetic composition of the present disclosure may be contained in an amount of 0.001 to 1 wt%, preferably 0.01 to 0.1 wt%, more preferably 0.05 to 0.5 wt%, based on the total weight of the composition,. In this content range, appropriate formulation stability may be ensured, and the desired antioxidant effect may be expected.

In addition to containing the active ingredient of the present disclosure, the composition of the present disclosure may further contain known natural extracts or other ingredients for antioxidant, skin aging prevention, and skin regeneration promotion effects within a range that does not inhibit the effective activity of the active ingredient of the present disclosure.

Furthermore, the cosmetic composition may contain components which are conventionally added to cosmetic compositions, for example, fatty substances, organic solvents, solubilizing agents, thickeners, gelling agents, softeners, antioxidants, suspending agents, stabilizers, foaming agents, aromatics, surfactants, water, ionic or nonionic emulsifying agents, fillers, sequestering agents, chelating agents, preservatives, vitamins, blockers, moisturizing agents, essential oil, dyes, pigments, and hydrophilic or hydrophobic activators, and may be formulated in any form using additives or carriers which are conventionally used in the cosmetic field or the skin science field.

The cosmetic composition of the present disclosure may be prepared as any formulation that is commonly prepared in the art, and for example, it may be formulated into a solution, suspension, emulsion, paste, gel, cream, lotion, powder, pack, soap, surfactant-containing cleanser, oil, spray, or the like, without being limited thereto. More specifically, the cosmetic composition may be prepared as formulations such as softening lotion, nourishing lotion, nourishing cream, massage cream, essence, eye cream, powder foundation, emulsion foundation, wax foundation, cleansing cream, cleansing foam, cleansing water, pack, spray, powder, pact, lip gloss, lipstick, shadow, shampoo, or rinse.

When the formulation of the present disclosure is a paste, cream or gel, a carrier component that may be used is animal oil, vegetable oil, wax, paraffin, starch, tragacanth, a cellulose derivative, polyethylene glycol, silicone, bentonite, silica, talc, zinc oxide, or the like.

When the formulation of the present disclosure is a powder or spray, a carrier component that may be used is lactose, talc, silica, aluminum hydroxide, calcium silicate or polyamide powder, and in particular, when the formulation is a spray, it may further contain a propellant such as chlorofluorohydrocarbon, propane/butane, or dimethyl ether.

When the formulation of the present disclosure is a solution or emulsion, a carrier component that may be used is a solvent, solubilizing agent or emulsifying agent, examples of which include water, ethanol, isopropanol, ethyl carbonate, ethyl acetate, benzyl alcohol, benzyl benzoate, propylene glycol, 1,3-butyl glycol oil, glycerol fatty acid esters, polyethylene glycol, or sorbitan fatty acid esters.

When the formulation of the present disclosure is a suspension, a carrier component that may be used is a liquid diluent such as water, ethanol or propylene glycol, a suspending agent such as ethoxylated isostearyl alcohol, polyoxyethylene sorbitol ester or polyoxyethylene sorbitan ester, microcrystalline cellulose, aluminum metahydroxide, bentonite, agar, tragacanth, or the like.

When the formulation of the present disclosure is a surfactant-containing cleanser, a carrier component that may be used is aliphatic alcohol sulfate, aliphatic alcohol ether sulfate, sulfosuccinic acid monoester, isethionate, an imidazolinium derivative, methyl taurate, sarcosinate, fatty acid amide ether sulfate, alkylamidobetaine, aliphatic alcohol, fatty acid glyceride, fatty acid diethanolamide, vegetable oil, a lanolin derivative, ethoxylated glycerol fatty acid ester, or the like.

The present disclosure also provides a reagent composition for inhibiting activation, proliferation or differentiation of T cells containing L-AHG.

The T cells are preferably CD4 T cells.

The activation refers to activation of resting T cells.

The proliferation refers to an increase in T-cell number by cell cycle progression.

The differentiation means differentiation into Th1 cells and Th2 cells.

The reagent composition may be applied to T cells under ex *vivo* conditions, preferably *in vitro* conditions, thereby inhibiting activation, proliferation and differentiation of the T cells.

The present disclosure also provides a method for inhibiting activation, proliferation or differentiation of T cells, preferably CD4 T cells, the method including treating T cells with L-AHG of the present disclosure under ex *vivo* conditions.

The term "*ex vivo* conditions" means that T cells are present in a state physically completely isolated from a living body. The *ex vivo* conditions are preferably *in vitro* conditions.

The T cells may be cells isolated from the blood of a subject, a subculture of the cells, or a cell line.

The treatment with L-AHG may be performed, for example, by adding L-AHG to a medium containing T cells in a cell culture dish, and the treatment concentration is 1.0 to 1,000 µg/mL, preferably 12.5 to 400 µg /mL.

The inhibition of differentiation may be inhibition of differentiation into Th1 and Th2 cells. The inhibition of differentiation into Th1 and Th2 cells may lead to a decrease in the secretion of interferon-gamma (INF-γ), interleukin-2 (IL-2), interleukin-4 (IL-4) or interleukin-13 (IL-13).

Hereinafter, the present disclosure will be described in more detail with reference to specific examples. The following examples describe preferred embodiments of the present disclosure, and the scope of the present disclosure is not construed as being limited by the matters described in the following examples.

### [Examples]

### 1. Materials and methods

### 1.1. Animals

5-6 week old C57BL/6J male mice were purchased from OrientBio (322 Galmachi-ro, Jungwon-gu, Seongnam-si, Gyeonggi-do, Korea) and used in experiments while being maintained under specific pathogen-free conditions at the Kyungpook National University-Laboratory Animal Resources Center (680 Gukchaebosang-ro, Jung-gu, Daegu-si, Korea).

### 1.2. Chemicals, antibodies, reagents and media

Mouse recombinant IL-2 (rIL-2) was purchased from Cambridge Bioscience (Cambridge, UK). Rabbit anti-mouse IgG was purchased from Jackson ImmunoResearch (West Grove, PA, USA), and anti-CD3 was purchased from BD Pharmingen (San Diego, CA, USA). Goat anti-mouse IL-2 antibody for neutralizing IL-2 activity was purchased from R&D System (Minneapolis, MN, USA), and rat anti-mouse IL-2R neutralizing antibody was purchased from Abcam (Cambridge, UK). The ECL Western Blotting Kit was purchased from Thermo Fisher Scientific (Rockford, IL, USA). Immobilon-P membranes for Western blotting analysis were purchased from EMD Millipore Corporation (Temecula, CA, USA). The microtubule depolymerization agent nocodazole and all other electrophoresis reagents were purchased from Sigma Chemical (St. Louis, MO, USA).

In order to obtain neoagarotetraose (NA4) and neoagarohexaose (NA6), which are the hydrolysis products of agarose, 9.0% agarose dissolved in water by heat treatment was hydrolyzed with GH16B β-agarase (1.6 µg/mL) under optimal reaction conditions (1 mM MnCl₂ and 10 mM TCEP, 50 mM Tris-HCl, pH 7.0, 50°C) for 14 hours while continuously stirring with a magnetic stirrer, thereby producing NA4 and NA6. The produced NA4/NA6 mixture was purified by fractionation by size-exclusion chromatography using a Sephadex G-15 column. In order to obtain neoagarobiose (NA2) from agarose, 9% agarose was first hydrolyzed into neoagarooligosaccharides (NAOSs) including NA4/NA6 by treatment with GH16B β-agarase at 50°C, and then the temperature was lowered to 35°C, and then GH50A β-agarase (20 µg/mL) was added thereto and allowed to react for 14 hours, thereby producing NA2. NA2 in the reaction product was purified by fractionation by size-exclusion chromatography using a Sephadex G-15 column. In addition, in order to obtain L-AHG, a constituting monomer of agarose, first, 9% agarose was first hydrolyzed into NAOSs including NA4 and NA6 by treatment with GH16B β-agarase at 50°C, and then the temperature was lowered to 35°C, and then GH50A β-agarase (20 µg/mL) and GH117A α-NABH (60 µg/mL) were simultaneously added thereto and allowed to react for 14 hours, thus obtaining a mixture of the monomers L-AHG and D-galactose as final products. For final purification of L-AHG from this mixture, the mixture was fractionated by size-exclusion chromatography using a Sephadex G-10 column.

The medium used for T lymphocyte activation and culture was RPMI 1640 containing 10% FBS (HyClone, Logan Utah, USA), 20 mM HEPES, 50 µM β-mercaptoethanol, and 100 ug/mL gentamycin, which was named complete medium.

### 1.3. Isolation, activation, and measurement of [³H]thymidine incorporation of resting (G0) T cells

Isolation of resting (G0) T lymphocytes from spleens from 4-5-month-old C57BL/6J male mice was performed by a negative selection method from splenocytes using a T cell purification column kit (R&D Systems, Minneapolis, Minn., USA) . At this time, the activation of G0 T cells was performed using a modification of the method of Kim et al., wherein pretreatment with rabbit anti-hamster IgG antibody (20 µg/mL) was performed so that the surface of the culture plastic container was smoothly coated with anti-CD3 (2.0 µg/mL) and anti-CD28 (2.0 µg/mL).

To evaluate the proliferation of T cells activated by immobilized anti-CD3/anti-CD28, 1 × 10⁵ G0 T cells and each concentration of L-AHG together with 200 µL of RPMI 1640 complete medium were added to each well of a 96-well plate coated with anti-CD3 (2.0 µg/mL) and anti-CD28 (2.0 µg/mL), and activation of the cells was initiated by culture. At 29 hours after the activation, 0.25 µCi of [³H]-thymidine was added to each well, and the amount of [³H]-thymidine incorporated into the DNA of proliferating T cells was quantified by a liquid scintillation counter while the cells were further cultured for 12 hours.

To analyze the cell cycle of T cells activated by immobilized anti-CD3/anti-CD28 or to analyze the cytokine concentration in the culture supernatant, 3 × 10⁶ G0 T cells and each concentration of L-AHG together with 2 mL of RPMI 1640 complete medium were added to each well of a 35-mm plate coated with anti-CD3 (2.0 µg/mL) and anti-CD28 (2.0 µg/mL), and activation of the cells was initiated by culture. At 30 hours after the activation, the culture was stopped and the cells and the culture supernatant were recovered by centrifugation.

Under the same culture conditions, in order for the first cell cycle progression initiated by the activation of G0 T cells to be arrested in prometaphase by nocodazole treatment, T lymphocytes were activated for 20 hours, and then nocodazole was added thereto at a concentration of 0.2 µg/mL, followed by followed by further culturing for an additional 10 hours. After completion of the culturing, the culture was centrifuged to recover the cells and the culture supernatant.

### 1.4. T cell surface staining with fluorescently labeled anti-CD4/anti-CD8 antibody and intracellular DNA staining with DAPI

Resting (G0) T cells isolated from mouse splenocytes using a T cell purification column kit (R & D) and T cells obtained by activating G0 T cells with immobilized anti-CD3/anti-CD28 for a given period of time were subjected to anti-CD3, anti-CD4, anti-CD8, and DAPI staining.

About 5 × 10⁵ cells were suspended in 100 µL of 1X PBS/1% FBS solution, and then 0.5 µg of anti-CD3, 0.5 µg of anti-CD4, and 0.5 µg of anti-CD8 were added thereto and allowed to react at 4°C for 15 minutes, followed by washing twice with 1X PBS/1 % FBS solution. Subsequently, the cells were fixed with 1% paraformaldehyde solution at 4°C for 15 minutes, and the fixed cells were washed twice with 1X PBS solution and then stained with 10 µM DAPI for 15 minutes. The stained cells were analyzed with an Attune NxT flow cytometer (Thermo Fisher Scientific, Waltham, MA, USA) to determine the proportions of CD3 T cells, CD4 T cells, and CD8 T cells and the cell cycle distribution based on the increase in blue fluorescence depending on the amount of DAPI bound to intracellular DNA.

### 1.5. Enzyme-linked immunosorbent assay (ELISA)

After recovering the culture supernatant of T cells, the concentrations of IFN-γ, IL-2, IL-4 and IL-13 therein were analyzed using an ELISA kit (Mouse Uncoated Elisa Kit, Invitrogen) according to the manufacturer's instructions. In summary, 100 ul of a capture antibody dilution was first added to each well of a 96-well plate and incubated at 4°C for 16 hours, followed by washing three times with a washing solution. Each well was blocked with 200 ul of blocking solution at 25°C for 1 hour and washed once with a washing solution. Subsequently, 100 ul of a solution in which the standard sample and the culture supernatant were appropriately diluted were added to each well and allowed to react at 25°C for 2 hours. After washing three times with a washing solution, 100 µl of secondary antibody (detection antibody) solution was added to each well and allowed to react at 25°C for 1 hour. After washing three times with a washing solution, 100 µl of an avidin-HRP solution was added to each well and allowed to react at 25°C for 30 minutes. After washing 5 times with a washing solution, 100 µl of a substrate solution was added to each well and allowed to react at 25°C for 15 minutes under a light-shielded condition. The reaction was terminated by adding 100 µl of a reaction stop solution to each well, and the absorbances at wavelengths of 450 nm and 570 nm were measured with a Varioskan Lux Multimode Microplate Reader (Thermo Fisher Scientific). At this time, the value obtained by subtracting the absorbance measured at a wavelength of 570 nm from the absorbance measured at a wavelength of 450 nm was compared with a standard curve to determine the concentrations of cytokines present in the culture supernatant. For preparation of the standard curve, serially diluted recombinant IFN-γ, IL-2, IL-4 and IL-13 were used as standard samples.

### 2. Results

### 2.1. Effect of enzymatic hydrolysates of agarose on [³H]-thymidine incorporation in G0 T cells stimulated by immobilized anti-CD3/anti-CD28

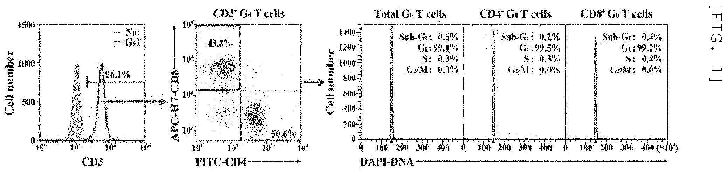

Isolation of resting (G0) lymphocytes from mouse spleens was performed by Percoll gradient centrifugation. G0 T lymphocytes were isolated from G0 lymphocytes by a negative selection method using a T cell purification column kit (R&D Systems, Minneapolis, MN, USA). As a result of staining the surfaces of the G0 T cells, isolated by the T cell purification column, using fluorescently labeled anti-CD3, anti-CD4, and anti-CD8 antibodies, 96% of the cells were identified as CD3+ T cells (FIG. 1). In addition, 50.6% of these CD3+ T cells were identified as CD4+ T (T_{H}) cells and 43.8% as CD8+ T (T_{C}) cells.

The isolated resting T cells were stimulated and stimulation with immobilized anti-CD3/CD28 in a 96-well plate. At this time, each of NA4 and NA6 obtained by hydrolyzing agarose with GH16B β-agarase, NA2 obtained by first hydrolysis of agarose with GH16B β-agarase and then further hydrolysis with GH50B β-agarase, and L-AHG obtained by first hydrolysis of agarose with GH16B β-agarase and then further hydrolysis with both GH50B β-agarase and GH117A α-NABH was added to the cells at a concentration of 12.5 to 400 µg/mL in order to investigate the effects of these agarose degradation products (L-AHG, NA2, NA4, and NA6) on cell cycle progression and proliferation following activation of the resting T cells.

First, using a 96-well plate, resting T cells (1 × 10⁵ G0 T cells/well) were stimulated and activated with immobilized anti-CD3/anti-CD28 in the presence of various concentrations (0, 12.5, 25, 50, 100, 200, and 400 µg/mL) of L-AHG, NA2, NA4, or NA6. At 29 hours after the activation, 0.25 µCi of [³H]thymidine was added to each well, followed by further culturing for an additional 12 hours. The amount of [³H]thymidine incorporated into DNA following the T cell proliferation that occurred during this additional culturing time was quantified with a liquid scintillation counter, thereby determining the degree of T cell proliferation that occurred in each well.

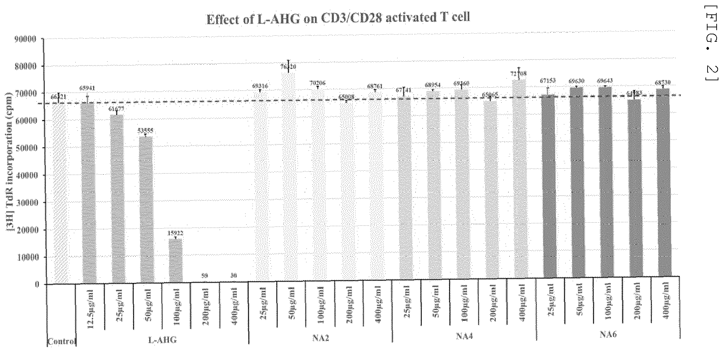

As a result, as shown in FIG. 2, it was confirmed that 12.5 ug/ml of L-AHG had no significant effect on the proliferation of activated T cells, and thus the level of [³H]thymidine incorporation at this L-AHG concentration was almost identical to that of the L-AHG-untreated control group. In contrast, the presence of 25 to 400 ug/mL of L-AHG inhibited [³H]thymidine incorporation in a concentration-dependent manner. In particular, it was shown that, in the presence of 100 ug/mL of L-AHG, [³H]thymidine incorporation decreased to about 22% of that in the L-AHG-untreated control group, and at an L-AHG concentration of 200 to 400 µg/mL, [³H]thymidine incorporation significantly decreased to an unmeasurable level. However, it was shown that agarose degradation products such as NA2, NA4, and NA6 under the same conditions had no significant effect on the [³H]thymidine incorporation of activated T cells at a concentration of 25 to 400 µg/mL.

From these results, it was confirmed that only L-AHG among the enzymatic hydrolysates of agarose investigated had the physiological activity of significantly inhibiting the proliferation of activated T cells.

Until recently, various physiological activities of agar degradation products (NA2, L-AHG, NAOS, and AOS) have been reported. That is, these physiological activities include antioxidant, prebiotic, antitumor, anti-inflammatory, antidiabetic, antiobesity, and skin moisturizing and whitening activities. In addition, it has been reported that oral administration of NA4 in a mouse model exhibits a protective effect against fatigue and liver damage, caused by strenuous exercise, through the regulation of intestinal microbiota. However, the inhibitory effect of L-AHG against the proliferation of activated T cells is a new physiological activity that has not been reported to date.

### 2.2. Effect of L-AHG on cell cycle progression of G0 T cells stimulated by immobilized anti-CD3/anti-CD28

T lymphocytes of the immune system can be roughly divided into two types: CD4 T (T_{H}) lymphocytes and CD8 T (T_{C}) lymphocytes. The inventors of the present disclosure examined whether the proliferation inhibitory activity of L-AHG by the inhibition of DNA synthesis in activated T cells as confirmed by the [³H]thymidine incorporation assay would occur commonly in CD4 T (T_{H}) cells and CD8 T (T_{C}) cells in common, or whether it would occur only in either of the two types of T cells.

To this end, resting T cells were stimulated and activated with immobilized anti-CD3/anti-CD28, and when these cells reached the time point at which S phase started following G0 exit and G1 entry and progression, that is, when 20 hours have elapsed after the activation, the microtubule depolymerization agent nocodazole (a cell cycle-arresting agent that arrests cell cycle progression of eukaryotic cells at the prometaphase of M phase) was added to the cells, followed by further culturing for 10 hours. Through this unique experimental method, the first cell cycle initiated following the activation of resting T cells was allowed to progress through the G1, S, and G2 phases, and then arrest in the prometaphase of M phase due to microtubule damage caused by the effect of nocodazole. Then, these cells were recovered, treated and stained with fluorescently labeled anti-CD4 antibody, fluorescently labeled anti-CD8 antibody, and the DNA fluorescent staining reagent DAPI, and analyzed with a flow cytometer. As a result, the present inventors investigated how the aspect of the first cell cycle progression (G1, S, and G2/M phases), which is initiated following the activation of resting CD4 T cells and resting CD8 T cells, is affected by L-AHG.

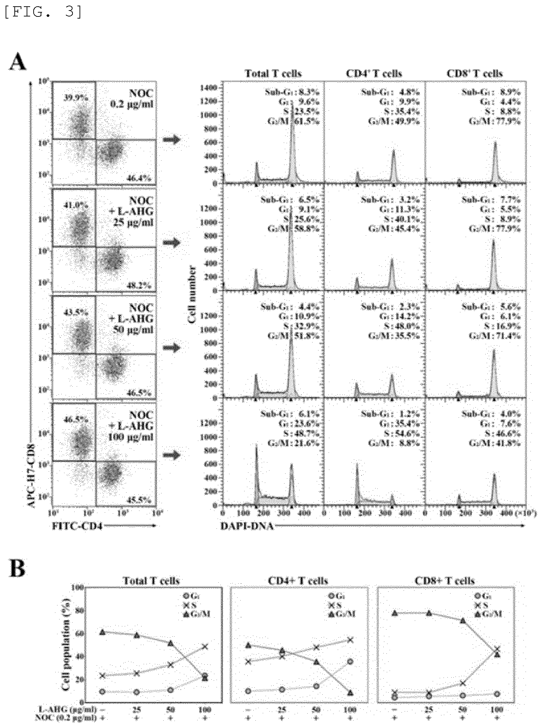

As shown in FIGS. 3A and 3B, as a result of analyzing the stained cells by flow cytometry, it was shown that, when resting T cells were stimulated and activated with immobilized anti-CD/anti-CD28, 4.4%, 8.8%, and 77.9% of CD8 T cells were distributed in G1, S, and G2/M phases, respectively, whereas 9.9%, 35.4%, and 49.9% of CD4 T cells were distributed in G1, S, and G2/M phases, respectively. That is, at 30 hours after the activation, the percentage of CD8 T cells remaining in the G1 + S phase without entering the G2/M phase was only about 13.2%, but the percentage of CD4 T cells remaining in the G1 + S phase was as high as about 45.3%. These results show that, among resting T cells, CD8 T cells progress through the cell cycle relatively more quickly and smoothly than to CD4 T cells after activation with immobilized anti-CD/anti-CD28.

The first cell cycle progressions following activation of resting CD4 T and CD8 T cells, identified as described above, were found to be affected significantly differently by the addition of L-AHG at a concentration of 25 to 100 ug/ml. That is, the percentage of resting CD4 T cells remaining in the G1 and S phases without entering the G2/M phase after activation with immobilized anti-CD3/anti-CD28 increased in a manner dependent on the concentration of L-AHG. In particular, it was shown that, when 100 ug/mL of L-AHG was added, the percentages of G1- and S-phase cells were 35.4% and 54.6%, respectively, while the percentage of G2/M phase cells was only 8.8%, suggesting that cell cycle progression was significantly inhibited by L-AHG. In contrast, cell cycle progression after activation of resting CD8 T cells was hardly affected by the addition of 25 to 50 ug/mL of L-AHG, and when 100 ug/mL of L-AHG was added, the percentage of S-phase cells increased to 46.6% and the percentage of G2/M cells was 41.8%, while the change in the percentage of G1-phase cells was negligible.

These findings indicate that cell cycle progression following activation of resting CD4 T cells was inhibited mainly in G1 and S phases in a concentration-dependent manner by the addition of L-AHG (25 to 100 µg/mL), and particularly, when 100 ug/ml of L-AHG was added, the percentage of cells remaining in the G1 + S phase without entering the G2/M phase was about 90.0% of the total cells. Under the same conditions, cell cycle progression following activation of resting CD8 T cells was not significantly affected by the addition of 25 to 50 ug/mL of L-AHG, and even when 100 ug/mL of L-AHG was added, the progression of the G1 phase was unaffected, but the progression of the S phase was somewhat inhibited. As such, it was confirmed that cell cycle progressions following activation of resting CD4 T and CD8 T cells were inhibited in different ways by the presence of L-AHG (25-100 µg/mL), but apoptotic cell death (based on the proportion of apoptotic sub-G1 cells) in these cells was not induced by L-AHG.

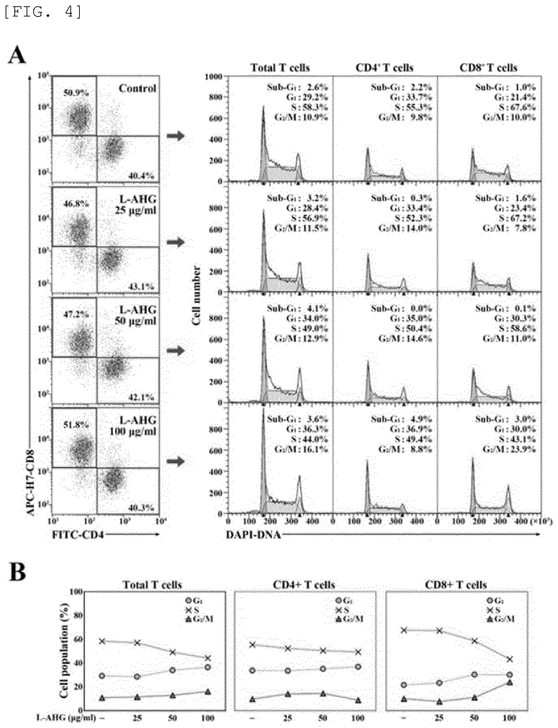

Meanwhile, it was shown that, when resting T cells were stimulated and activated with immobilized anti-CD3/anti-CD28 under the same conditions and cultured for 30 hours without the addition of nocodazole, it was not easy to confirm the inhibitory effect of L-AHG on cell cycle progression following activation of resting CD4 T and CD8 T cells by flow cytometry (FIGS. 4A and 4B). This is believed to be because the cell cycle progression initiated by activation of resting CD4 T cells or resting CD8 T cells is inhibited by the presence of L-AHG in some cells, but is not inhibited by L-AHG in the other cells, and these other cells progress to the G1, S, and G2 phases after completion of the first cell cycle progression, and eventually, cells whose cell cycle progression has been inhibited by L-AHG and cells whose cell cycle progression has occurred even in the presence of L-AHG are mixed together and co-exist.

Taken together, the present study results suggest that the inhibitory effect of L-AHG on the proliferation of activated T cells mainly targets CD4 T cells, not CD8 T cells, and inhibits the cell cycle progression of CD4 T cells in G1 phase and S phase.

### 2.3. Effect of L-AHG on cytokine production in G0 T cells stimulated by immobilized anti-CD3/anti-CD28

Unlike CD8 T cells, CD4 T cells differentiate into several functionally differentiated effector CD4 T cell subtypes and modulate various immune responses. Th1, Th2, Th17, Tfh and Treg cells are known as effector CD4 T cell subtypes. These subtypes can be distinguished from one another based on the characteristics of the cytokine combinations they secrete.

Th1 cells produce IFN-γ and promote the activation of macrophages, thereby enabling more efficient destruction of microorganisms capable of surviving or multiplying in macrophages. Th2 cells are involved in recruiting and activating eosinophils (IL-5) and mast cells/basophils (IL-4) by producing cytokines such as IL-4, IL-5, and IL-13, and also enhance the barrier immunity of the mucosal surface (IL-13). They contribute to controlling infection by parasites by promoting a defense reaction mainly through IgE. In particular, IL-4 and IL-13 are also required for class switching of B cells to produce IgE. IgE produced by B cells with the help of Th2 cells in this way may cause hypersensitivity reactions such as allergy and asthma. Th17 cells secrete IL-17A, IL17F, and IL-22, which induce local epithelial and stromal cells to produce chemokines that recruit neutrophils to the site of infection. Thereby, they amplify the reaction of neutrophils to remove pathogens such as extracellular bacteria and fungi. In addition, they activate mucosal epithelial tissue and skin epithelial cells to produce antibacterial peptides that attack bacteria. T_{FH} cells are an effector CD4 T cell subtype that benefits B cells in lymphoid tissue. T_{FH} cells form an interactive relationship with naive B cells through mechanisms such as traffic to B cell follicles and linked recognition of antigens, and promote germinal center responses, helping in antibody production and class switching of B cells. T_{FH} cells produce a large amount of the cytokine IL-21 that supports the proliferation of B cells and their differentiation into antibody-producing plasma cells. Treg cells usually suppress T cell activity and innate immune cell activity through the production of TGF-β and IL-10, thus limiting the immune response, and help prevent autoimmunity from developing during the immune response.

The stabilities of effector CD4 T cell subtypes differ from each other. Naive CD4 T cells have various potentials, whereas Th1 and Th2 cells show a relatively stable ground state and are highly resistant to conversion to other subtypes. Meanwhile, iTreg cells and Th17 cells are less stable and can be converted to other subtypes by cytokines that predominantly affect. iTreg cells can be converted to Th17 cells in response to IL-6 and IL-1, and become Th1 cells in response to IL-12. Th17 cells can also be converted to Th1 cells in response to IL-12. Here, it is understood that the conversion of iTreg cells to Th17 cells and the conversion of Th17 cells to Th1 cells is unidirectional or irreversible.

Based on the differentiated immune-related functions of effector CD4 T cell subtypes, it will be more effective to use an immunosuppressant exerting a specific inhibitory ability that targets Th1 and Th2, among effector CD4 T cell the subtypes, in order to control an inappropriate immune response, which occurs in hypersensitivity patients, autoimmune disease patients, and organ transplant patients including stem cell or bone marrow transplant patients, to a necessary level.

It was shown that L-AHG (25 to 100 µg/mL) significantly inhibited the proliferation of activated CD4 T cells by inhibiting cell cycle progression of activated CD4 T cells mainly in G1 and S phases in a concentration-dependent manner.

At 30 hours after the activation of resting T cells by stimulation with immobilized anti-CD3/ant-CD28 under the same conditions, the culture supernatant was recovered, and the concentrations of the Th1 signature cytokines (IFN-γ and IL-2) known to be secreted by Th1 cells and the Th2 signature cytokines (L-4 and IL-13) known to be secreted by Th2 cells were measured by an ELISA method.

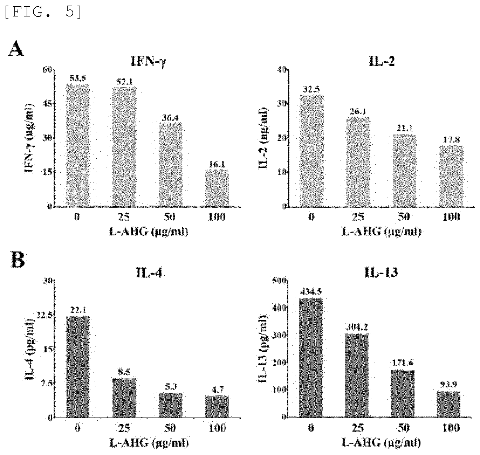

As a result, it was shown that not only IL-2 and IFN-γ secreted by the Th1 subtype, but also IL-4 and IL-13 secreted by the Th2 subtype, contained in the culture supernatant of activated T cells, were significantly decreased by the addition of L-AHG (25, 50, and 100 µg/mL) in a concentration-dependent manner (FIGS. 5A and B). Interestingly, a more pronounced decrease in the concentrations of the Th2 signature cytokines compared to the concentrations of the Th1 signature cytokines was observed. In particular, it was confirmed that, in the presence of L-AHG at a concentration of 25 µg/mL, the IFN-γ concentration was hardly affected, but the IL-4 concentration significantly decreased to 38.5% of that in the L-AHG-untreated control group.

In conclusion, the above results suggest that, when clonal expansion and differentiation into effector Th1 and Th2 subtypes following cell proliferation occur as a result of activating resting Th cells by stimulation with immobilized anti-CD3/ant-CD28 to initiate the cell cycle, the presence of L-AHG can inhibit differentiation into Th1 and Th2 subtypes by inhibiting cell cycle progression of CD4 T cells, and can more strongly inhibit differentiation into the Th2 subtype than the Th1 subtype.

### [Deposit of Microorganisms]

Depository authority: the Korean Collection for Type Cultures (KCTC) at the Korea Research Institute of Bioscience and Biotechnology
Accession number: KCTC13629BP
Deposit date: August 27, 2018

## Claims

1. An immunosuppressive composition containing 3,6-anhydro-L-galactose (L-AHG) as an active ingredient.

2. The immunosuppressive composition of claim 1, which inhibits activation, proliferation or differentiation of CD4 T cells.

3. The immunosuppressive composition of claim 2, which blocks G1 or S phase cell cycle progression.

4. The immunosuppressive composition of claim 1, which inhibits differentiation into Th1 cells or Th2 cells.

5. The immunosuppressive composition of claim 4, which inhibits production of interferon-gamma (INF-γ), interleukin-2 (IL-2), interleukin-4 (IL-4), or interleukin-13 (IL-13).

6. A pharmaceutical composition for preventing or treating immunological disease containing 3,6-anhydro-L-galactose (L-AHG) as an active ingredient.

7. The pharmaceutical composition of claim 6, wherein the immunological disease is selected from the group consisting of hypersensitivity disease, autoimmune disease, immunorejection, graft-versus-host disease, Sjogren's syndrome, and Behcet's disease.

8. The pharmaceutical composition of claim 7, wherein the hypersensitivity disease is selected from the group consisting of allergy, sneezing, wheezing, asthma, atopic dermatitis, and hives.

9. The pharmaceutical composition of claim 7, wherein the autoimmune disease is selected from the group consisting of multiple sclerosis, rheumatoid arthritis, systemic lupus erythematosus, type 1 diabetes, psoriasis, Hashimoto's thyroiditis, systemic lupus erythematosus, and acute inflammatory bowel disease.

10. A health functional food for preventing or ameliorating immunological diseases containing 3,6-anhydro-L-galactose (L-AHG) as an active ingredient.

11. The health functional food of claim 10, wherein the immunological disease is selected from the group consisting of hypersensitivity disease, autoimmune disease, immunorejection, graft-versus-host disease, Sjogren's syndrome, and Behcet's disease.

12. The health functional food of claim 11, wherein the hypersensitivity disease is selected from the group consisting of allergy, sneezing, wheezing, asthma, atopic dermatitis, and hives.

13. The health functional food of claim 11, wherein the autoimmune disease is selected from the group consisting of multiple sclerosis, rheumatoid arthritis, systemic lupus erythematosus, type 1 diabetes, psoriasis, Hashimoto's thyroiditis, systemic lupus erythematosus, and acute inflammatory bowel disease.

14. A functional cosmetic composition for preventing or ameliorating hypersensitivity skin reactions containing 3,6-anhydro-L-galactose (L-AHG) as an active ingredient.

15. The functional cosmetic composition of claim 14, wherein the hypersensitivity skin reaction is selected from the group consisting of solar dermatitis, contact dermatitis, and atopic dermatitis.
